(19) **Europäisches Patentamt / European Patent Office / Office européen des brevets**

(11) **EP 4 060 315 A1**

(12) # EUROPEAN PATENT APPLICATION
published in accordance with Art. 153(4) EPC

(43) Date of publication:
**21.09.2022 Bulletin 2022/38**

(21) Application number: **20902229.2**

(22) Date of filing: **13.11.2020**

(51) International Patent Classification (IPC):
***G01N 15/02*** *(2006.01)*

(86) International application number:
**PCT/CN2020/128740**

(87) International publication number:
**WO 2021/120939 (24.06.2021 Gazette 2021/25)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **20.12.2019 CN 201911326511**

(71) Applicant: **Resun (Shenzhen) Tech Co., Ltd.**
**Shenzhen, Guangdong 518000 (CN)**

(72) Inventors:
• **WANG, Zhe**
  **Shenzhen, Guangdong 518000 (CN)**
• **LIU, Ke**
  **Shenzhen, Guangdong 518000 (CN)**
• **XIONG, Gui**
  **Shenzhen, Guangdong 518000 (CN)**

(74) Representative: **Russell, Tim et al**
  **Venner Shipley LLP**
  **200 Aldersgate**
  **London EC1A 4HD (GB)**

(54) **MICRO-NANO PARTICLE DETECTION DEVICE AND METHOD**

(57)    A micro-nano particle detection device and method are disclosed. The device includes a sample chamber (101) and at least two measurement chambers (102), where at least one through hole is formed between each measurement chamber (102) and the sample chamber (101), each measurement chamber (102) is communicated with the sample chamber (101) only through the through hole, a common electrode (G) is arranged in the sample chamber (101), a measurement electrode (A to F) is arranged in each measurement chamber (102) respectively, a first end of the sample chamber (101) is provided with a first liquid driving device (M), and the common electrode (G) is grounded. In the detection device, a plurality of measurement chambers (102) are provided, and the through hole is arranged between each measurement chamber (102) and the sample chamber (101), so that a diameter and a concentration of micro-nano particles passing through the through holes can be detected, thus effectively solving the problem that particle size distribution of micro-nano particles in a sample solution with wide particle size distribution cannot be measured. The detection device can be widely used in the field of micro-nano particle detection.

FIG. 1

EP 4 060 315 A1

## Description

### TECHNICAL FIELD

The disclosure relates to the technical field of micro-nano particle detection, and more particularly, to a micro-nano particle detection device and method.

### BACKGROUND

Particle size is used as an important physical parameter. Based on its special properties, the particulate matter is widely used in medicine, chemical industry, materials and other fields. In application of the particulate matter, it is very important to measure particle size distribution and other properties of a micro-nano particulate matter (with a particle size ranging from 1 nm to 10 $\mu$m, hereinafter referred to as micro-nano particles).

At present, commonly used particle measurement devices include optical microscope, scanning electron microscope and transmission electron microscope. However, due to a low resolution of the optical microscopes, it is difficult to observe particles with a size less than 300 nm, thus being not suitable for measuring the micro-nano particles.

The particle size can be observed through the scanning electron microscope and the transmission electron microscope, but particle size distribution of a sample cannot be fully and accurately represented due to a limited field of vision. Moreover, real morphological information cannot be obtained for a biological particle sample or a particle sample required to be measured in a solution state.

By a dynamic light scattering method of a Malvern particle size analyzer, large-scale particle detection can be quickly carried out, but features of individual particles cannot be extracted, mixed particles cannot be distinguished and identified, and particle size distribution of a sample with wide particle size distribution cannot be given accurately.

Coulter's principle is a classic particle size measurement method, and when particles in electrolyte pass through a small hole tube with the electrolyte, the electrolyte with the same volume is replaced, so that a resistance between two electrodes inside and outside the small hole tube is changed instantaneously in a circuit designed with a constant current, and a potential pulse is produced to measure a volume of the particles. However, the Coulter's principle is limited by an diameter of a small hole (too big particles are easy to block the hole, and too small particles results in too weak signals to be measured), so that a sample with too wide particle size distribution cannot be measured.

Therefore, there is still a problem in the existing technology that the particle size distribution of the micro-nano particles in the sample solution with wide particle size distribution cannot be measured.

## SUMMARY

In order to solve the above technical problem, the disclosure aims to provide a micro-nano particle detection device and method.

In a first aspect, a micro-nano particle detection device is provided, which includes a sample chamber and at least two measurement chambers, where at least one through hole is provided between each measurement chamber and the sample chamber, each measurement chamber is communicated with the sample chamber only through the through hole, a common electrode is arranged in the sample chamber, each measurement chamber is provided with a measurement electrode respectively, a first end of the sample chamber is provided with a first liquid driving device, and the common electrode is grounded.

In a possible implementation, the sample chamber is provided with a sample tube close to the first end of the sample chamber, a first end of the sample tube is provided with a second liquid driving device, and a second end of the sample tube is arranged in a center of the first end of the sample chamber.

In a possible implementation, a diameter of the through hole ranges from 10 nm to 10 um.

In a possible implementation, diameters of the through holes corresponding to a same measurement chamber are the same, and diameters of the through holes corresponding to different measurement chambers are different.

In a possible implementation, the diameters of the through holes are set from small to large along a liquid flow direction.

In a possible implementation, the diameters of the through holes are set in a gradient way.

In a possible implementation, voltages or currents of the measurement electrodes are set from small to large along a liquid flow direction.

In a possible implementation, the voltages or the currents of the measurement electrodes are set in a gradient way.

In a second aspect, a detection method applied to the micro-nano particle detection device is provided, which includes:

applying a voltage or a current to the measurement electrode;

outputting an electrolyte sample containing to-be-detected micro-nano particle liquid through the first liquid driving device;

detecting a stable current value H of the measurement electrode before the micro-nano particles enter the through hole and a maximum current change h after the micro-nano particles enter the through hole;

according to the stable current value H and the maximum current change h, obtaining an equivalent volume of the micro-nano particles; and

according to the equivalent volume, obtaining an equivalent diameter of the micro-nano particles.

In a possible implementation, the detection method further includes:

counting a total number of the micro-nano particles in all the measurement chambers; and

according to the total number of the micro-nano particles and a sample volume of the electrolyte sample added, obtaining a concentration of the to-be-detected micro-nano particle liquid.

The disclosure has the beneficial effects as follows.

According to the micro-nano particle detection device and method of the disclosure, a plurality of measurement chambers are provided, and the through hole is arranged between each measurement chamber and the sample chamber, so that a diameter and a concentration of micro-nano particles passing through the through holes can be detected, thus effectively solving the problem that particle size distribution of micro-nano particles in a sample solution with wide particle size distribution cannot be measured.

**BRIEF DESCRIPTION OF THE DRAWINGS**

FIG. 1 is a schematic structural diagram of a micro-nano particle detection device according to an embodiment of the disclosure;

FIG. 2 is a schematic structural diagram of a micro-nano particle detection device according to another embodiment of the disclosure;

FIG. 3 is a schematic diagram of an electric field in a micro-nano particle detection device according to an embodiment of the disclosure; and

FIG. 4 is a flow chart of a detection method of the disclosure.

**DETAILED DESCRIPTION**

The specific implementations of the disclosure are further described hereinafter with reference to the drawings.

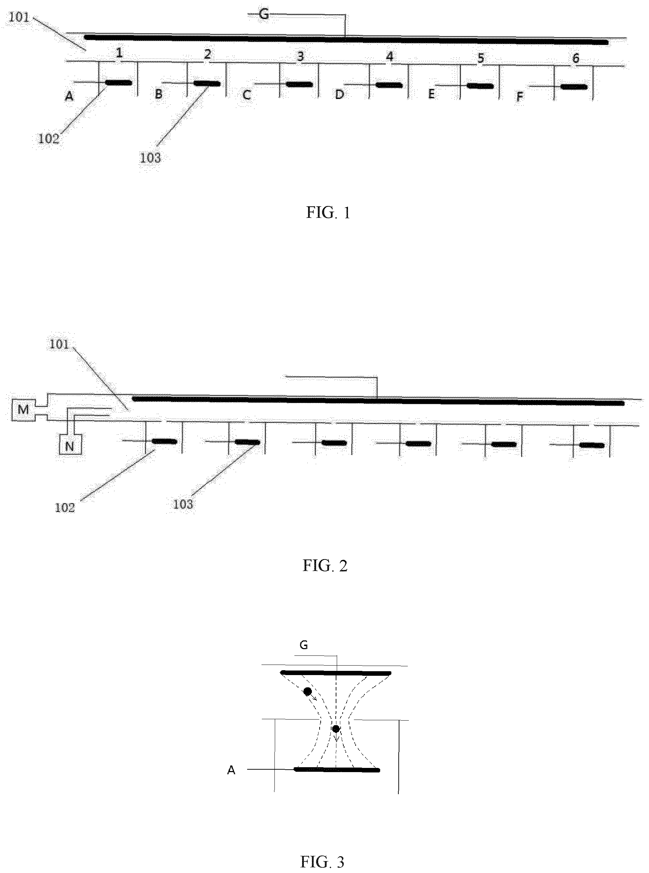

With reference to FIG. 1, a micro-nano particle detection device is provided according to an embodiment, which includes a sample chamber 101 and at least two measurement chambers 102. At least one through hole is provided between each measurement chamber 102 and the sample chamber 101, each measurement chamber 102 is communicated with the sample chamber 101 only through the through hole, a common electrode G is arranged in the sample chamber 101, each measurement chamber 102 is provided with a measurement electrode 103 respectively, a first end of the sample chamber 101 is provided with a first liquid driving device, and the common electrode G is grounded.

In this embodiment, a voltage or a current is applied to each measurement electrode 103 during detection, and then the sample chamber 101 and the measurement chambers 102 are filled with an electrolyte sample by the first liquid driving device. The electrolyte sample contains to-be-detected micro-nano particle liquid, and the electrolyte sample flows from the first end of the sample chamber 101 to the other end under an action of the first liquid driving device. When to-be-detected micro-nano particles enter the measurement chambers 102 from the sample chamber 101 through the through hole, size information of a single micro-nano particle is obtained by detecting a change of the voltage or the current on the measurement electrode 103, thus obtaining a concentration and particle size distribution of the sample.

With reference to FIG. 1, the sample chamber 101 in this embodiment is realized by a capillary tube, the sample chamber 101 is the capillary tube with a diameter of 10 um, the sample chamber 101 is communicated with 6 measurement chambers 102 through 6 microholes, diameters of the 6 microholes are 100 nm, 200 nm, 400 nm, 800 nm, 1,600 nm and 3,200 nm in sequence, and a hole depth is 500 nm. One common electrode G is provided in the sample chamber 101, and the measurement electrodes 103 of the measurement chambers 102 are an electrode A, an electrode B, an electrode C, an electrode D, an electrode E and an electrode F in sequence. The sample chamber 101 and the measurement chambers 102 are filled with the electrolyte sample, where the common electrode G is grounded. Voltages of 0.2 V, 0.4 V, 0.8 V, 1.5 V, 3.3 V and 6.5 V are applied to the measurement electrodes A to F in sequence. As shown in FIG. 3, the electric field is formed between the microhole and the electrode.

Then, the electrolyte containing to-be-detected polyethylene microsphere particles is pushed into the sample chamber 101 from a left end by the first liquid driving device, a particle size of the polyethylene microsphere particles ranges from 30 nm to 1,000 nm, and a Zeta potential on surfaces of the polyethylene microsphere particles is negative. When passing through the electric field area between the microhole and the common electrode G, the polyethylene microsphere particles are deviated under an action of an electric field force, move towards the microhole, and finally enter the measurement chamber 102 through the microhole under the action of the electric field force.

When the microsphere particles do not enter the microhole, the current will keep at a stable current value H. When the micro-nano particles enter the microhole, the current will reduce greatly. Due to Coulter's principle, a maximum current change h is proportional to an equivalent volume V of the micro-nano particles. The equivalent volume V may be calculated by the following formula:

$$V=a*h/H;$$

where a is a proportional coefficient, which is related to a size parameter of the microhole, and a value of a may be determined by an experiment.

Then, an equivalent diameter may be obtained through the equivalent volume. When cube equivalence is performed, a third power of the equivalent diameter is the equivalent volume, and the equivalent diameter may be calculated through the volume.

The higher the voltage applied to the measurement electrode 103 is, the larger an electric field strength will be, and the stronger the electric field force will be. When a micro-nano particle swarm with different particle sizes flows from a left end to a right end of the sample chamber 101 along with the electrolyte, the micro-nano particle swarm may be deviated under the action of the electric field force while passing through a vicinity of the microhole. When the micro-nano particle swarm passes through a vicinity of microhole No. 1, small particles are pulled to pass through the microhole No. 1 by the electric field force. At the moment, since the electric field force is weak, large particles cannot be pulled, and the large particles may flow to microhole No. 2 under driving of the electrolyte. By analogy, at a position closer to the right end, the voltage of the measurement chamber 102 is higher, the action of the electric field force is stronger, and the particles capable of being pulled are larger, so that the particles passing through the microhole are larger at the position closer to the right end. Finally, all the particles reach the measurement chambers 102 through different microholes, thus realizing division of the micro-nano particles with different particle sizes, and realizing accurate measurement of the particle sizes of the micro-nano particles with different diameters by the microholes with different diameters.

By counting a total number N of the micro-nano particles detected in all measurement chambers 102, a concentration of a to-be-detected sample may be calculated as p=N/VA, where VA is a volume of the sample added into the sample chamber 101.

With reference to FIG. 2, in a possible implementation, the sample chamber 101 is provided with a sample tube close to the first end, a first end of the sample tube is provided with a second liquid driving device N, and a second end of the sample tube is arranged in a center of the first end of the sample chamber 101.

In this embodiment, the sample chamber 101 is a capillary tube with a diameter of 10 um, and one end of the sample chamber 101 is provided with a first liquid driving device M. There is a sample tube with a diameter of 2um at the same end, one end of the sample tube is provided with the second liquid driving device N, and the other end of the sample tube is located in a center of the sample chamber 101. The first liquid driving device M outputs electrolyte, and the second liquid driving device N outputs electrolyte containing to-be-detected micro-nano particles. Electrolyte output speeds of M and N are (10X10-2X2): (2X2) = 24: 1, so that a stable sheath flow can be formed in the sample chamber 101, and the to-be-detected particles may move to the right in the center of the sample chamber 101, thus having an advantage that deviation discrimination of the particles of different diameters can be increased when the to-be-detected micro-nano particles are deviated by the electric field force.

In a possible implementation, a diameter of the through hole ranges from 10 nm to 10 um.

In a possible implementation, diameters of the through holes corresponding to the same measurement chamber 102 are the same, and diameters of the through holes corresponding to different measurement chambers 102 are different.

In a possible implementation, the diameter of the through hole is set from small to large along a liquid flow direction. The diameters of the through holes in the same measurement chamber 102 are still the same, and the setting from small to large herein is for the through holes corresponding to different measurement chambers 102.

In a possible implementation, the diameter of each through hole is set in a gradient way. The diameter may be set in equal difference or equal ratio.

In a possible implementation, a voltage or a current of each measurement electrode 103 is set from small to large along a liquid flow direction.

In a possible implementation, the voltage or the current of each measurement electrode 103 is set in a gradient way. The voltage or the current may be set in equal difference or equal ratio.

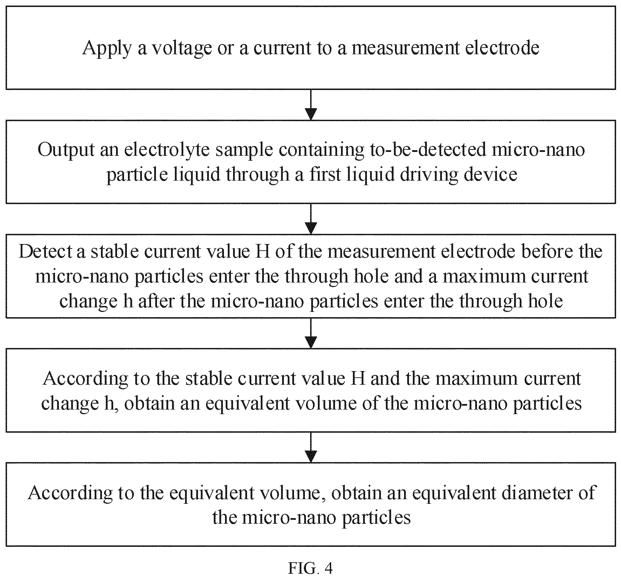

With reference to FIG. 4, a detection method applied to the micro-nano particle detection device is provided, which includes:

applying a voltage or a current to the measurement electrode 103;

outputting an electrolyte sample containing to-be-detected micro-nano particle liquid through the first liquid driving device;

detecting a stable current value H of the measurement electrode 103 before the micro-nano particles enter the through hole and a maximum current change h after the micro-nano particles enter the through hole;

according to the stable current value H and the maximum current change h, obtaining an equivalent volume of the micro-nano particles; and

according to the equivalent volume, obtaining an equivalent diameter of the micro-nano particles.

In this embodiment, when the microsphere particles do not enter the microhole, the current will keep at a stable current value H. When the micro-nano particles enter the microhole, the current will reduce greatly. Due to Coulter's principle, a maximum current change h is proportional to an equivalent volume V of the micro-nano particles. The equivalent volume V may be calculated by the following formula:

$$V = a*h/H;$$

where a is a proportional coefficient, which is related to a size parameter of the microhole, and a value of a may be determined by an experiment.

Then, an equivalent diameter may be obtained through the equivalent volume. When cube equivalence is performed, a third power of the equivalent diameter is the equivalent volume, and the equivalent diameter may be calculated through the volume.

In a possible implementation, the detection method further includes:

counting a total number of the micro-nano particles in all the measurement chambers 102; and

according to the total number of the micro-nano particles and a sample volume of an electrolyte sample added, obtaining a concentration of the to-be-detected micro-nano particle liquid.

In this embodiment, by counting a total number N of the micro-nano particles detected in all measurement chambers 102, a concentration of a to-be-detected sample may be calculated as $p = N/VA$, where VA is a volume of the sample added into the sample chamber 101.

It can be seen from the above contents that in the disclosure, a plurality of measurement chambers 102 are provided, and the through hole is arranged between each measurement chamber 102 and the sample chamber 101, so that a diameter and a concentration of micro-nano particles passing through the through holes can be detected, thus effectively solving the problem that particle size distribution of micro-nano particles in a sample solution with wide particle size distribution cannot be measured.

The foregoing describes some embodiments of the disclosure in detail, but the disclosure is not limited to the embodiments. Those skilled in the art may further make various equivalent modifications or substitutions without violating the gist of the disclosure, and these equivalent modifications or substitutions are all included in the scope defined by the claims of the present application.

## Claims

1. A micro-nano particle detection device, comprising a sample chamber and at least two measurement chambers, wherein at least one through hole is provided between each measurement chamber and the sample chamber, each measurement chamber is communicated with the sample chamber only through the through hole, a common electrode is arranged in the sample chamber, each measurement chamber is provided with a measurement electrode respectively, a first end of the sample chamber is provided with a first liquid driving device, and the common electrode is grounded.

2. The micro-nano particle detection device of claim 1, wherein the sample chamber is provided with a sample tube close to the first end of the sample chamber, a first end of the sample tube is provided with a second liquid driving device, and a second end of the sample tube is arranged in a center of the first end of the sample chamber.

3. The micro-nano particle detection device of claim 1, wherein a diameter of the through hole ranges from 10 nm to 10 um.

4. The micro-nano particle detection device of claim 1, wherein diameters of the through holes corresponding to a same measurement chamber are the same, and diameters of the through holes corresponding to different measurement chambers are different.

5. The micro-nano particle detection device of claim 4, wherein the diameters of the through holes are set from small to large along a liquid flow direction.

6. The micro-nano particle detection device of claim 5, wherein the diameters of the through holes are set in a gradient way.

7. The micro-nano particle detection device of claim 1, wherein voltages or currents of the measurement electrodes are set from small to large along a liquid flow direction.

8. The micro-nano particle detection device of claim 7, wherein the voltages or the currents of the measurement electrodes are set in a gradient way.

9. A detection method applied to the micro-nano particle detection device of any one of claims 1 to 8, comprising:

applying a voltage or a current to the measurement electrode;
outputting an electrolyte sample containing to-be-detected micro-nano particle liquid through the first liquid driving device;
detecting a stable current value H of the measurement electrode before the micro-nano particles enter the through hole and a maximum current change h after the micro-nano particles enter the through hole;
according to the stable current value H and the maximum current change h, obtaining an equivalent volume of the micro-nano particles; and
according to the equivalent volume, obtaining an equivalent diameter of the micro-nano particles.

10. The detection method of claim 9, further comprising:

counting a total number of the micro-nano particles in all the measurement chambers; and
according to the total number of the micro-nano particles and a sample volume of the electrolyte sample added, obtaining a concentration of the to-be-detected micro-nano particle liquid.

FIG. 1

FIG. 2

FIG. 3

Apply a voltage or a current to a measurement electrode

Output an electrolyte sample containing to-be-detected micro-nano particle liquid through a first liquid driving device

Detect a stable current value H of the measurement electrode before the micro-nano particles enter the through hole and a maximum current change h after the micro-nano particles enter the through hole

According to the stable current value H and the maximum current change h, obtain an equivalent volume of the micro-nano particles

According to the equivalent volume, obtain an equivalent diameter of the micro-nano particles

FIG. 4

## INTERNATIONAL SEARCH REPORT

| International application No. |
| --- |
| **PCT/CN2020/128740** |

| A. | CLASSIFICATION OF SUBJECT MATTER |
| --- | --- |
| | G01N 15/02(2006.01)i |

According to International Patent Classification (IPC) or to both national classification and IPC

| B. | FIELDS SEARCHED |
| --- | --- |

Minimum documentation searched (classification system followed by classification symbols)

G01N

Documentation searched other than minimum documentation to the extent that such documents are included in the fields searched

Electronic data base consulted during the international search (name of data base and, where practicable, search terms used)

CNPAT, EPODOC, WPI, CNKI: 微纳, 微米, 纳米, 颗粒, 粒子, 粒径, 直径, 尺寸, 分布, 通孔, 库尔特, 电极, 电流, 电压, 脉冲, 计数; micro+, nano+, particle?, diameter, size, distribution, orifice?, hole?, coulter, electrode?, count+, pulse?, current, voltage

| C. | DOCUMENTS CONSIDERED TO BE RELEVANT |
| --- | --- |

| Category* | Citation of document, with indication, where appropriate, of the relevant passages | Relevant to claim No. |
| --- | --- | --- |
| PX | CN 111122398 A (RUIXIN ZHIZAO (SHENZHEN) TECHNOLOGY CO., LTD.) 08 May 2020 (2020-05-08) description, paragraphs [0009]-[0025], and figures 1-4 | 1-10 |
| PX | CN 211453257 U (RUIXIN ZHIZAO (SHENZHEN) TECHNOLOGY CO., LTD.) 08 September 2020 (2020-09-08) description, paragraphs [0009]-[0018], and figures 1-3 | 1-10 |
| Y | CN 209570489 U (GUANGDONG PHARMACEUTICAL UNIVERSITY) 01 November 2019 (2019-11-01) description, paragraphs [0003], [0014] and [0022]-[0027], and figures 1-2 | 1-10 |
| Y | CN 105738623 A (TOSHIBA K.K.) 06 July 2016 (2016-07-06) description paragraphs [0046]- [0068], figures 1-4 | 1-10 |
| A | CN 107340226 A (JIANGSU UNIVERSITY) 10 November 2017 (2017-11-10) entire document | 1-10 |
| A | CN 108732080 A (TAIZHOU UNIVERSITY) 02 November 2018 (2018-11-02) entire document | 1-10 |
| A | US 2007159156 A1 (HU, Jun et al.) 12 July 2007 (2007-07-12) entire document | 1-10 |

☐ Further documents are listed in the continuation of Box C.   ☑ See patent family annex.

| * | Special categories of cited documents: | "T" | later document published after the international filing date or priority date and not in conflict with the application but cited to understand the principle or theory underlying the invention |
| --- | --- | --- | --- |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
| --- | --- |
| **11 January 2021** | **29 January 2021** |

| Name and mailing address of the ISA/CN | Authorized officer |
| --- | --- |
| **China National Intellectual Property Administration (ISA/CN)** **No. 6, Xitucheng Road, Jimenqiao, Haidian District, Beijing 100088 China** | |
| Facsimile No. **(86-10)62019451** | Telephone No. |

Form PCT/ISA/210 (second sheet) (January 2015)

INTERNATIONAL SEARCH REPORT
Information on patent family members

International application No.

**PCT/CN2020/128740**

| Patent document cited in search report | | | Publication date (day/month/year) | Patent family member(s) | | | Publication date (day/month/year) |
|---|---|---|---|---|---|---|---|
| CN | 111122398 | A | 08 May 2020 | None | | | |
| CN | 211453257 | U | 08 September 2020 | None | | | |
| CN | 209570489 | U | 01 November 2019 | None | | | |
| CN | 105738623 | A | 06 July 2016 | CN | 105738623 | B | 24 April 2018 |
| | | | | US | 2016187335 | A1 | 30 June 2016 |
| | | | | US | 9804116 | B2 | 31 October 2017 |
| | | | | JP | 2016126003 | A | 11 July 2016 |
| | | | | JP | 6495806 | B2 | 03 April 2019 |
| CN | 107340226 | A | 10 November 2017 | None | | | |
| CN | 108732080 | A | 02 November 2018 | None | | | |
| US | 2007159156 | A1 | 12 July 2007 | US | 7777476 | B2 | 17 August 2010 |
| | | | | US | 7397232 | B2 | 08 July 2008 |
| | | | | US | 2009066315 | A1 | 12 March 2009 |

Form PCT/ISA/210 (patent family annex) (January 2015)